Europäisches Patentamt

European Patent Office

Office européen des brevets

(19)

(11) Numéro de publication: **0 306 421**
**A1**

(12) **DEMANDE DE BREVET EUROPEEN**

(21) Numéro de dépôt: 88420297.9

(22) Date de dépôt: 01.09.88

(51) Int. Cl.⁴: **A 61 K 37/26**
A 61 K 9/18, A 61 K 9/54

(30) Priorité: 02.09.87 FR 8712709

(43) Date de publication de la demande:
08.03.89 Bulletin 89/10

(84) Etats contractants désignés:
AT BE CH DE ES FR GB GR IT LI LU NL SE

(71) Demandeur: MEDIBREVEX, Société Civile dite
1 avenue Marcelin Berthelot
F-38100 Grenoble (FR)

(72) Inventeur: Ayache, Josiane
3 boulevard Joffre
F-38000 Grenoble (FR)

Ayache, Jean-Jacques
3 boulevard Joffre
F-38000 Grenoble (FR)

Bruttmann, Georges
11 Place Victor Hugo
F-38000 Grenoble (FR)

Pedrali, Patrick
Chemin des Chapelaines La Boutaevilloise
F-74000 Annecy (FR)

Robert, Serge
14 rue des Dêgues
B-1440 Braine le Chateau (BE)

(74) Mandataire: Maureau, Philippe et al
Cabinet Germain & Maureau Le Britannia - Tour C 20, bld
Eugène Déruelle Boîte Postale 3011
F-69392 Lyon Cédex 03 (FR)

(54) Nouvelles formes galéniques d'insuline pour administration par voie per- et sublinguale.

(57) Selon l'invention, l'insuline, sous forme "rapide", "semi-retard" ou "retard", est contenue en quantités strictement contrôlées et reproductibles dans des supports solides prévus pour une libération progressive du principe actif par voie per- et sublinguale.

Le procédé de préparation de ces nouvelles formes galéniques comporte les étapes suivantes :
- mise en solution d'insuline dans un solvant polaire pour obtenir une solution-mère ;
- préparation de dilutions à différentes concentrations,
- fractionnement de chacune de ces dilutions en sous-dilutions,
- imprégnation par multi-imprégnation ou imprégnation fractionnée d'un support solide pharmaceutiquement acceptable avec chacune des sous-dilutions, chacune desdites étapes d'imprégnation étant suivie d'un séchage à air forcé et desséché à une température inférieure ou égale à 30°C ;
- imprégnation finale protectrice du type dragéification.

EP 0 306 421 A1

## Description

## NOUVELLES FORMES GALENIQUES D'INSULINE POUR ADMINISTRATION PAR VOIE PER- ET SUBLINGUALE

La présente invention concerne de nouvelles formes galéniques d'insuline pour administration par voie per- et sublinguale.

Le diabète insulino-dépendant est traité dans le monde entier par des injections sous cutanées d'insuline.

Ces injections peuvent se faire de une à trois fois par jour, selon l'équilibre glycémique.

Dans les cas graves, qui sont devenus rares (comas hyperglycémiques avec acide-cétose), on doit recourir à un traitement par voie intraveineuse et une rééquilibration hydro-électrolytique en milieu spécialisé.

Ces injections répétées dans la journée et durant toute la vie, sont une astreinte énorme pour le diabétique qui doit, avant chaque injection, vérifier le taux de sa glycémie : il s'agit de l'auto-contrôle. Après avoir prélevé une goutte de sang au bout du doigt, une bandelette réactive est imprégnée : le taux de la glycémie est obtenu en comparant la coloration de la bandelette à une échelle colorimétrique. La dose d'insuline nécessaire est alors préparée dans une seringue spéciale. L'absence ou la présence de sucre dans les urines, contrôlée avec une bandelette différente, est un reflet grossier de la glycémie.

Devant toutes ces obligations incontournables et astreignantes, des études ont été réalisées pour tenter de trouver une nouvelle forme galénique de l'insuline (rapide, semi-retardée, retardée), qui éviterait au malade l'astreinte de la piqûre avec tout le matériel que cela implique.

C'est ainsi que l'on a essayé d'appliquer l'insuline par voie nasale (gouttes et aérosols), par voie buccale, par voie rectale.

Dans tous les cas, l'efficacité a été démontrée par une baisse de la glycémie chez les diabétiques et chez les témoins, mais les formes galéniques utilisées présentent des inconvénients qui n'en ont pas permis l'exploitation industrielle :

- la forme destinée à l'administration par voie nasale est imprécise ou mal tolérée (liquide mis en gouttes nasales et en aérosols) ;

- la forme destinée à l'administration par voie buccale est dégradée par les sucs digestifs car l'insuline est une hormone protéique qu'elle soit d'origine humaine ou animale;

- la forme destinée à l'administration par voie rectale est mal tolérée, car il est nécessaire de placer plusieurs suppositoires par jour.

Toutes ces nouvelles formes galéniques ont une activité hypo-glycémiante certaine mais ne permettent pas d'administrer des doses fiables, telles qu'elles sont délivrées par les seringues.

La présente invention s'est donné pour objet de proposer de nouvelles formes galéniques d'insuline évitant au malade l'astreinte de la piqûre sans pour autant présenter les inconvénients mentionnés ci-avant.

Les nouvelles formes galéniques d'insuline selon l'invention sont caractérisées en ce que l'insuline est contenue en quantités strictement contrôlées et reproductibles dans des supports solides prévus pour une libération progressive du principe actif par voie per - et sublinguale.

L'administration d'insuline par voie sublinguale s'est, en effet, révélé de façon surprenante aussi active que l'administration par voie injectable.

La forme sublinguale solide offre, de plus, l'avantage de présenter une imprégnation en insuline stable, dosable, reproductible, donc fiable d'un lot à l'autre, qu'il s'agisse de la forme insuline "rapide", "semi-retard" ou "retard" ou toute autre forme.

Cette voie per- et sublinguale est utilisée à cause de sa configuration anatomique qui en fait une entité par rapport aux autres éléments de la cavité buccale.

Schématiquement, on peut décrire la région sublinguale de la manière suivante (chacun des éléments constitutifs ayant des propriétés utiles dans l'administration des médicaments introduits par cette voie).

Globalement, la région sublinguale comprend:
- la face inférieure de la langue qui sert de plafond, très riche en vaisseaux sanguins : veines, artères et vaisseaux lymphatiques ;
- le plancher de la bouche qui constitue la partie inférieure de la région sublinguale. C'est également une région anatomique qui comporte beaucoup de vaisseaux sanguins et surtout un très riche réseau veineux, des artères et des vaisseaux lymphatiques ;
- les bords de la région sublinguale sont formés par les parties montantes du maxillaire inférieur, les gencives et les dents.

Dans le plancher de la bouche, on trouve des glandes salivaires, les glandes sublinguales, qui secrètent de la salive dès qu'il existe un contact avec la région sublinguale. Par sa composition, la salive participe activement au délitement du produit pharmacologiquement actif présenté sous cette forme galénique sublinguale particulière selon l'invention.

Par ailleurs, la structure histologique montre l'existence de cellules immuno-compétentes en grand nombre qui vont, dans certains cas, favoriser l'activité médicamenteuse.

Par rapport aux autres constituants de la bouche : intérieur des joues, partie supérieure de la langue, la région sublinguale apparaît donc comme une entité anatomique particulière qui en fait une voie d'administration médicamenteuse privilégiée comparable à l'administration des médicaments par voie injectable.

Tous les autres médicaments dont le délitement se fait dans la bouche sont forcément avalés.

Dans la forme galénique sublinguale selon l'invention, l'activité du produit est fondée sur sa possibilité d'absorption rapide sans déglutition. L'avantage que présente cette forme galénique par rapport aux autres formes galéniques utilisées à l'intérieur de la cavité buccale est que le produit passe directement dans le sang, ce qui évite le métabolisme hépatique ;

le produit agit donc plus rapidement avec une efficacité maximale.

Les autres avantages de la forme galénique sublinguale solide, par rapport à la voie injectable, sont les suivants :

- suppression des injections, souvent pénibles, douloureuses et traumatisantes du point de vue psychique, à tel point que certains malades ressentent ces injections comme une auto-mutilation ;
- suppression du "petit matériel", seringues, etc...
- arrêt de la dépendance totale du diabétique pour les injections,
- suppression des complications cutanées aux points d'injection (lypodystrophies).

Les nouvelles formes galéniques d'insuline selon l'invention sont d'un emploi très simple, sans contrainte pour le malade ; on peut proposer de donner le matin, avant les collations de dix et de seize heures, avant le repas de midi, quelques micro-comprimés ou granules d'insuline "rapide" pour éviter les pics d'hyperglycémie. Cela présente l'avantage, par rapport aux injections, de ne pas nécessiter dans la journée d'apport d'insuline lente ou retard.

Par contre, pour couvrir les besoins nocturnes, il faudra donner des granules d'insuline lente ou retard, soit avant le repas du soir, en même temps que la "rapide", soit au coucher.

Les nouvelles formes galéniques selon l'invention offrent également, en cas de besoin, la possibilité de prises supplémentaires diurnes en ambulatoire.

On notera, en outre, la facilité de transport des nouvelles formes galéniques d'insuline selon l'invention, que l'on peut préparer sous conditionnement type "blister" qu'il suffit de conserver au frais.

Les formes galéniques sublinguales solides selon l'invention ne sont destinées qu'aux traitements ambulatoires ; elles ne sont, en aucun cas, prévues pour des malades nécessitant des soins urgents en milieu spécialisé.

Le procédé d'obtention des nouvelles formes galéniques d'insuline selon l'invention va maintenant être expliqué en détail.

Le produit de départ est l'insuline brute, ou une solution d'insuline à 40 UI/ml, ou un lyophilisat obtenu à partir de cette dernière solution.

Dans les deux premiers cas, l'insuline est remise en solution dans du sérum physiologique, afin d'obtenir la concentration voulue, exprimée en UI/ml.

L'imprégnation est réalisée sur un support qui peut être :
- des globules constitués d'un mélange de sorbitol et de lactose,
- des micro-comprimés sublinguaux dont les excipients sont du mannitol et/ou du lactose et/ou du sorbitol ;
- une poudre de mannitol mise en gélules par la suite.

Les doses d'insuline par gélule de globules, par poudre ou par micro-comprimés, seront comprises entre 1 UI et 10 UI, qu'il s'agisse d'une insuline rapide, semi-retard ou retard ou toute autre forme d'insuline.

Toutes les précautions sont prises, lors de chacune de ces dilutions ou sous-dilutions, pour que la quantité de solvant porteur d'insuline reste constante, ceci afin de réaliser toutes les imprégnations de façon identique.

On réalise ensuite, dans une turbine, à l'aide d'un injecteur du type dénommé "spray-doseur", l'imprégnation à l'aide de chacune des dilutions ou sous-dilutions de globules constitués, de façon connue en soi, du support solide pharmaceutiquement acceptable.

La technique d'imprégnation est celle de la multi-imprégnation ou de l'imprégnation fractionnée, de manière à garantir une parfaite répartition du principe actif homogène.

Entre chaque imprégnation, le séchage est réalisé par passage d'air forcé et desséché (à température inférieure à 30°C) dans une chambre où règne une dépression obtenue par la différence de débit existant entre ce passage d'air et un puissant extracteur.

La dernière imprégnation est protectrice, du type "dragéification". Une protection supplémentaire garantissant la totale conservation de l'intégrité physico-chimique des substances peut, si nécessaire, être apportée à la technique générale d'imprégnation ci-dessus en travaillant en atmosphère azotée.

La dernière opération consiste à mettre en gélules les globules ainsi obtenus, ou éventuellement à placer lesdits globules ou les poudres ou comprimés dans des tubes-doses et à terminer le conditionnement de façon classique (blisters pour les gélules, coffrets pour les tubes-doses) en numérotant, bien évidemment, les gélules à doses croissantes.

**Revendications**

1- Nouvelles formes galéniques d'insuline, caractérisées en ce que l'insuline est contenue en quantités strictement contrôlées et reproductibles dans des supports solides prévus pour une libération progressive du principe actif par voie per- et sublinguale.

2- Nouvelles formes galéniques d'insuline selon la revendication 1, caractérisées en ce que l'insuline se trouve sous une forme rapide, semi-retard ou retard.

3- Nouvelles formes galéniques d'insuline selon la revendication 1 et la revendication 2, caractérisées en ce que les doses d'insuline sont comprises entre 1 UI et 10 UI.

4- Procédé de préparation de formes galéniques solides d'insuline selon l'une quelconque des revendications 1 à 3, caractérisé par les étapes suivantes :
- mise en solution d'insuline dans un solvant polaire pour obtenir une solution mère,
- préparation de dilutions à différentes concentrations,
- fractionnement de chacune de ces dilutions en sous-dilutions,
- imprégnation par multi-imprégnation ou imprégnation fractionnée d'un support solide pharmaceutiquement acceptable avec chacune des sous-dilutions, chacune desdites étapes d'imprégnation étant suivie d'un séchage à air forcé et desséché à une température inférieure ou égale à 30°C ;
- imprégnation finale protectrice du type dragéification.

5- Procédé de préparation de formes galéniques solides d'insuline selon la revendication 4, caractérisé en ce que l'insuline se trouve sous forme de solution à 40 UI/ml.

6- Procédé selon la revendication 5, caractérisé en ce que l'insuline se trouve sous forme lyophilisée.

7- Procédé selon l'une quelconque des revendications 4 à 6, caractérisé en ce que le solvant polaire est le sérum physiologique.

8- Procédé selon l'une quelconque des revendications 4 à 7, caractérisé en ce que les différentes étapes de l'imprégnation sont effectuées sous atmosphère d'azote.

Office européen
des brevets

RAPPORT DE RECHERCHE EUROPEENNE

Numero de la demande

EP 88 42 0297

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | CHEMICAL ABSTRACTS, vol. 82, no. 14, 7 avril 1975, page 259, no. 90070t, Columbus, Ohio, US; & JP-A-74 17 563 (TAKEDA CHEMICAL INDUSTRIES, LTD) 01-05-1974 * Résumé * | 1-3 | A 61 K 37/26<br>A 61 K 9/18<br>A 61 K 9/54 |
| Y | IDEM | 4-8 | |
| Y | G. NETIEN et al.: "GALENICA 16 – MEDICAMENTS HOMEOPATHIQUES", 2ème édition, 1986, pages 91-99, Paris, FR * Pages 91-99 * | 4-8 | |
| X | AU-B- 419 560 (SISTER MARY PHILOMENA EARLE) * En entier * | 1-3 | |
| A | EP-A-0 127 535 (HADASSAH MEDICAL ORGANIZATION) * Page 15, exemples 20,21 * | | |
| A | GB-A-1 279 214 (FOREST LABORATORIES) * Page 4, exemple 8; revendications 1-4 * | | |

DOMAINES TECHNIQUES RECHERCHES (Int. Cl.4)

A 61 K

Le présent rapport a été établi pour toutes les revendications

| Lieu de la recherche | Date d'achèvement de la recherche | Examinateur |
|---|---|---|
| LA HAYE | 29-11-1988 | BENZ K.F. |